# EUROPEAN PATENT APPLICATION

(11) **EP 4 427 702 A1**
(43) Date of publication of application: **11.09.2024**
(21) Application number: 24153017.9
(22) Date of filing: 28.02.2023
(51) Int. Cl.: A61C 13/00, G16H 30/00

(54) **SYSTEMS AND METHODS FOR PROVIDING PERSONALIZED VIRTUAL DIGITAL DENTITION OF A PATIENT**

(62) Divisional of application: 23159268.4
(71) Applicant: Digicuto, 13008 Marseille (FR)
(72) Inventor: Koubi, Stefan, 13008 Marseille (FR); Gurel, Galip, 13008 Marseille (FR); Tourbah, Karim, 13008 Marseille (FR)
(74) Representative: A.P.I. Conseil

(57) **Abstract**

The subject application provides systems for providing personalized virtual digital dentition of a patient.

Indeed, inventors have found a way of designing a personalized virtual digital dentition of a patient that is based only on a digital image of the front face of the patient.

The system (100) comprises at least one memory (110), acquiring means (120) and computer vision means (130), which are operatively coupled to each other.

Computer vision means (130) may implement a machine learning classifier which is trained for predicting the likelihood that a set of cranio-facial features of a human face is similar to each of a set of reference cranio-facial features of a plurality of predetermined reference cranio-facial vectors.

## Description

### Technical Field

The subject application relates to systems for providing personalized virtual digital dentition of a patient.

### Background Art

It is known that facial attractiveness influences personality development and social interaction.

Among others, the smile of an individual is considered as an important element of facial attractiveness.

Principles of a pleasing smile have been described in the dental literature by numerous authors who suggest various parameters for optimal facial and dental composition.

Recently, it has been proposed to establish, for individuals, a relationship between human personality trait and dentofacial aesthetics.

Indeed, it is believed that as personality is unique to an individual, it could be used to design a customized smile.

However, today, human personality traits are determined by questionnaires that are time consuming to administer and process.

It is an object of the present subject application to provide a novel technique to overcome drawbacks typically associated with the use of questionnaires in the design of a customized smile.

### Summary of Subject application

The subject application provides systems for providing personalized virtual digital dentition of a patient, as described in the accompanying claims.

Dependent claims describe specific embodiments of the subject application.

These and other aspects of the subject application will be apparent from an elucidated based on the embodiments described hereinafter.

### Brief Description of Drawings

Further details, aspects and embodiments of the subject application will be described, by way of example only, with reference to the drawings. In the drawings, like reference numbers are used to identify like or functionally similar elements. Elements in the figures are illustrated for simplicity and clarity and have not necessarily been drawn to scale.
Figure 1 shows a block diagram of a system according to the subject application.
Figure 2 shows a segmented incisor, according to a first embodiment.
Figure 3 shows a segmented incisor, according to a second embodiment.
Figure 4 shows a schematic flow diagram of method according to the subject application.

### Description of Embodiments

Because the illustrated embodiments of the subject application may, for the most part, be composed of components known to the skilled person, details will not be explained in any greater extent than that considered necessary for the understanding and appreciation of the underlying concepts of the subject application, in order not to obfuscate or distract the reader from the teachings of the subject application.

Inventors have found a way of designing a personalized virtual digital dentition of a patient that is based only on a digital image of the front face of the patient.

The subject application relates to a system implemented by a computer for providing a personalized virtual digital dentition of a patient.

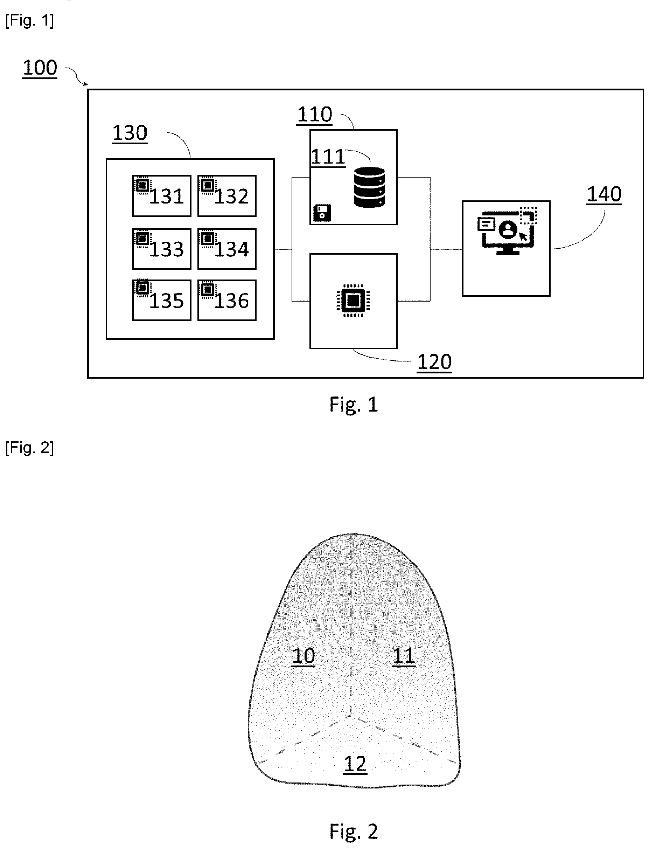

Referring to figure 1, the system 100 comprises at least one memory 110, acquiring means 120 and computer vision means 130, which are operatively coupled to each other.

In other words, one should understand that the system 100 may comprise more than one memory 110.

The memory 110 is of known type (i.e., any suitable computer-accessible or non-transitory storage medium for storing computer program instructions, such RAM, SDRAM, DDR SDRAM, RDRAM, SRAM, ROM, magnetic media, optical media and the like) and therefore will not be further detailed.

In the subject application, the memory 110 is configured for storing computer program instructions for execution by a processor.

The memory 110 is further configured for storing a plurality of predetermined reference cranio-facial vectors.

As used herein, the term 'vector' is defined as being any suitable data structure that holds a number of consecutive data elements.

In particular, each predetermined reference cranio-facial vector is associated with at least one type of predetermined human personality trait.

As used herein, the term 'human personality trait' is defined as personal characteristics of an individual that are revealed in a particular pattern of behavior in a variety of situations.

For instance, in the dentistry field, it is commonly admitted that an individual's personality is formed by a unique combination of four types of traits:
- 'choleric/strong' corresponding to individuals characterized by strong leadership qualities and fearlessness. They have a rectangular face with well-defined angles. The maxillary anterior teeth are positioned with their long axes perpendicular to the horizontal plane. These individuals have dominant rectangular central incisors. The connection line of the embrasures is horizontal between the central and lateral incisors,
- 'sanguine/dynamic' corresponding to individuals that are very active, communicative, and extroverted. They have an angular face. The long axes of the maxillary anterior teeth are inclined slightly distally. The central incisors are usually triangular or trapezoidal. The connection lines of the embrasures and the incisal plane are ascendants from the medial line,
- `melancholic/delicate' corresponding to individuals characterized by gentleness and abstract thinking. This type of individual has an oval face with rounded features. The long axes of the maxillary anterior teeth are distally inclined. The central incisors are usually oval. The connection lines of the embrasures descend from the medial line, creating an inverted incisal plane, and
- `phlegmatic/calm' corresponding to individuals that are gentle, discreet and diplomatic. They have a round or a square face. The maxillary anterior teeth have their long axes perpendicular to the horizontal plane. The central incisors are square and small. The connection line of the embrasures is straight.

However, other taxonomy of individual's personality traits may be contemplated, without requiring any substantial modification of the subject application.

In particular, one could consider combining reference cranio-facial vectors associated with different human personality traits to create combined reference cranio-facial vectors.

In practice, the personality trait of an individual can be determined through the use of a questionnaire (e.g., self-administered, researcher-administered or doctor-administered) that asks individuals to mark some statements that describe their own behavior.

However, other methods for determining the personal trait of an individual may be contemplated, without requiring any substantial modification of the subject application.

Also, each predetermined reference cranio-facial vector comprises a set of reference cranio-facial features.

As used herein, the term 'cranio-facial features' is defined as being any measurable characteristic that can be derived from a substantially front face view of an individual.

In an example, cranio-facial features comprise location and geometry (e.g., contours, angles, sizes) of anatomical parts of the face of an individual, such as the nose, the mouth, the eyes, the pupils, the eyebrows, the chin, the cheeks, and the forehead.

However, other anatomical parts or combination of anatomical parts of a front face of an individual may be contemplated, without requiring any substantial modification of the subject application.

In the subject application, the memory 110 is further configured for storing a teeth database 111.

As used herein, the term 'database' is defined as being any suitable data storage system such as a relational database (e.g., an object-relational database), a triple store, a hierarchical data store, or any suitable combination thereof.

The teeth database 111 comprises 3D digital images of individual teeth.

In particular, each 3D digital image is associated with one type of tooth (i.e., incisor, canine, premolar, molar) and at least one predetermined human personality trait.

In other words, one should understand that each 3D digital image may be associated with one type of tooth and more than one predetermined human personality trait.

Therefore, in the database 111, each data record corresponds to a single 3D digital image of an individual tooth which is associated with one type of tooth and at least one predetermined human personality trait.

In a first embodiment of the database 111, each data record corresponds to a single 3D digital image of an individual tooth which is associated with one type of tooth and one predetermined human personality trait.

In a second embodiment of the database 111, each data record corresponds to a single 3D digital image of an individual tooth which is associated with one type of tooth and more than one predetermined human personality trait.

This second embodiment aims at producing different tooth anatomies that extend beyond the standard tooth shapes.

In particular, in that second embodiment, each data record corresponds to a 3D digital image which is a combination of segmented portions of 3D digital images respectively associated with one predetermined human personality trait.

Indeed, each 3D digital image associated with one type of tooth and one predetermined human personality trait (such as those of the first embodiment of the database 111) may be segmented into two or more portions. Then, the segmented portions associated with one type of tooth and a plurality of predetermined human personality traits can be combined to form a combined 3D digital image. Therefore, the combined 3D digital image will be associated with the plurality of predetermined human personality traits.

Let's take an example with an incisor type of tooth.

In a first variation of the example, as illustrated in figure 2 which shows a section through a 3D digital image representing an incisor, each 3D digital image associated with an incisor can be segmented into three portions such as a mesial portion 10, a distal portion 11 and an incisal portion 12.

In that first variation of the example, for instance, a combined 3D digital image can correspond to a combination of a mesial portion 10 associated with a 'choleric/strong' human personality trait, a distal portion 11 associated with a 'sanguine/dynamic' human personality trait and an incisal portion 12 associated with a 'melancholic/delicate' human personality trait.

However, other combinations may be contemplated, without requiring any substantial modification of the subject application.

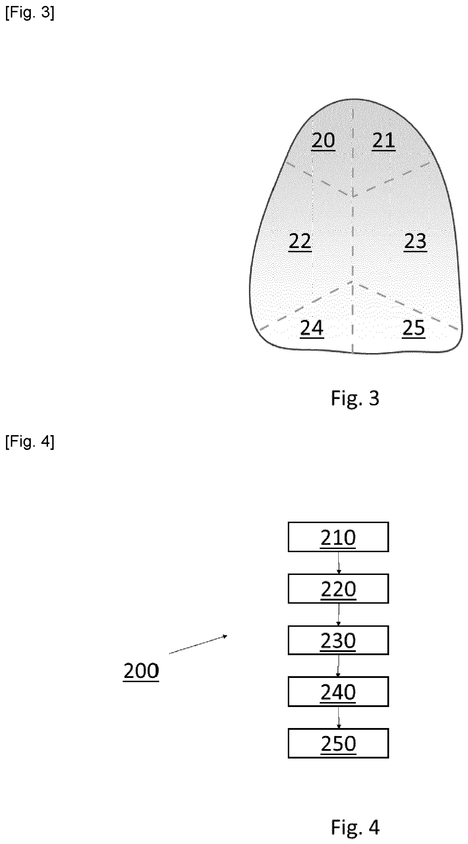

In a second variation of the example, as illustrated in figure 3 which shows a section through a 3D digital image representing an incisor, each 3D digital image associated with an incisor can be segmented into six portions such as a mesial cervical portion 20, a distal cervical portion 21, a mesial body portion 22, a distal body portion 23, a mesial incisal portion 24 and a distal incisal portion 25.

In that second variation of the example, for instance, a combined 3D digital image can correspond to a combination of a mesial cervical segmented portion 20 associated with a 'phlegmatic/calm' human personality trait, a distal cervical segmented portion 21 associated with a 'phlegmatic/calm' human personality trait, a mesial body segmented portion 22 associated with a `choleric/strong' human personality trait, a distal body segmented portion 23 associated with a 'choleric/strong' human personality trait, a mesial incisal segmented portion 24 associated with a 'sanguine/dynamic' human personality trait and a distal incisal segmented portion 25 associated with a 'sanguine/dynamic' human personality trait.

However, other combinations may be contemplated, without requiring any substantial modification of the subject application.

In a variation of the first and second embodiments of the database 111, each data record may also be associated with at least one particular visual characteristic of the single 3D digital image, such as the color or the tint of the tooth.

However, other measurable characteristics of 3D digital images may be contemplated, without requiring any substantial modification of the subject application.

In the subject application, the memory 110 is further configured for storing a first predetermined sequence.

In particular, the first predetermined sequence is representative of a type of tooth order, in which 3D digital images of individual teeth are to be considered for positioning on a 2D image.

As used herein, the term 'type of tooth order' is defined as corresponding to an order in which things occur based on the type of tooth.

In a first embodiment of the first predetermined sequence, the first predetermined sequence is ordered according to the following series: incisors, canines, premolars and molars.

In a second embodiment of the first predetermined sequence, the first predetermined sequence is ordered according to the following series: canines, incisors, premolars and molars.

However, other sequencing of the teeth according to their type may be contemplated, without requiring any substantial modification of the subject application.

The acquiring means 120 is of known type (i.e., any suitable processor capable of executing memory-stored instructions, such as a microprocessor, uniprocessor, a multiprocessor, and the like) and therefore will not be further detailed.

Still in the subject application, the acquiring means 120 is configured for acquiring at least one digital image of the front face of the patient smiling.

In other words, one should understand that the acquiring means 120 may acquire more than one digital image.

In a first implementation of the acquiring means 120, the acquired digital image is a 2D digital image.

In a second implementation of the acquiring means 120, the acquired digital image is a 2D digital representation of a section through a 3D digital image.

Further, the acquired digital image is such that at least a portion of the dentition of the patient can be seen.

In other words, one should understand that the acquired digital image may exhibit a substantial part of the dentition of the patient.

Yet, in the subject application, the computer vision means 130 comprises teeth detecting means 131, cranio-facial detecting means 132, ranking means 134, positioning means 135 and comparing means 133, which are operatively coupled to each other.

The computer vision means 130 is of known type (i.e., any suitable processor capable of executing memory-stored instructions implementing computer vision techniques, such as a microprocessor, uniprocessor, a multiprocessor, and the like) and therefore will not be further detailed.

The teeth detecting means 131 are configured for detecting at least one tooth in the digital image of the front face of the patient.

In other words, one should understand that the teeth detecting means 131 may detect more than one tooth in the digital image of the front face of the patient.

In an embodiment of the teeth detecting means 131, the teeth detecting means 131 are further configured for detecting a mouth opening boundary region formed between the upper lip, the lower lip, the oral commissures of the mouth of the patient.

Further, in the embodiment of the teeth detecting means 131, the teeth detecting means 131 detect the tooth within the mouth opening boundary region.

In an implementation of the embodiment of the teeth detecting means 131, the system 100 further comprises a user interface 140.

As used herein, the term 'user interface' is defined as being any suitable software rendering system for providing to and/or receiving information from a user of the user interface via a display device. The user interface may be based on one more of the following interactions: visual, graphical, tactile, audible, sensory or the like.

In the subject application, the user interface 140 is configured for allowing, through user interaction, digital modification of at least one point defining the mouth opening boundary region.

In other words, one should understand that the user interface 140 may allow digital modification of more than one point defining the mouth opening boundary region.

In the subject application, the cranio-facial detecting means 132 are configured for detecting a set of cranio-facial features of the patient based on the digital image of the front face of the patient.

The cranio-facial detecting means 132 are further configured for generating a patient cranio-facial vector based on the detected set of cranio-facial features.

In the subject application, the comparing means 133 are configured for comparing the patient cranio-facial vector with the plurality of predetermined reference cranio-facial vectors.

The comparing means 133 are further configured for calculating a plurality of similarity measures that are greater than a predetermined similarity threshold.

In particular, a similarity measure is representative of a matching similarity between the patient cranio-facial vector and one predetermined reference cranio-facial vector.

In other words, the number or similarity measures depends on the number of predetermined reference cranio-facial vectors.

In a first example, where there are two predetermined reference cranio-facial vectors, the comparing means 133 calculates two similarity measures.

In a second example, where there are four predetermined reference cranio-facial vectors, the comparing means 133 calculates four similarity measures.

In a first particular implementation of the comparing means 133, the similarity measures correspond to matching percentages.

In a second particular implementation of the comparing means 133, the comparing means 133 keeps only the similarity measures that are greater than a predetermined similarity threshold and discards the others.

In an example of the second particular implementation of the comparing means 133, in relation to the first particular implementation of the comparing means 133, the predetermined similarity threshold is greater than 0%, preferably greater than 2%, more preferably greater than 5%, most preferably greater than 10%.

In the subject application, the ranking means 134 are configured for ranking the similarity measures according to a given ranking order.

In a first embodiment of the ranking means 134, the given ranking order is an order from smaller to larger order.

In a second embodiment of the ranking means 134, the given ranking order is an order from larger to smaller order.

However, other ranking orders may be contemplated, without requiring any substantial modification of the subject application.

In the subject application, the positioning means 135 are configured for overlaying, on the digital image of the front face of the patient, the detected tooth with a 2D digital representation of a section through the corresponding 3D digital image of individual tooth, thereby generating a first personalized virtual 2D digital dentition of the patient.

In other words, one should understand that the positioning means 135 may overlay of more than one 2D digital representation of a section through a 3D digital image of individual tooth.

In particular, the overlaying is based on the first predetermined sequence and the ranked similarity measures.

In practice, the positioning means 135 determine, according to the first predetermined sequence, in which order the overlaying will be performed.

Further, the positioning means 135 selects, from the teeth database 111, at least one 3D digital image of individual tooth based on the ranked similarity measures.

Finally, the positioning means 135 overlay the detected tooth with the 2D digital representation of a section through the selected 3D digital image of individual tooth.

In order to illustrate the functioning of the positioning means 135, let's take an example in which,
- the first predetermined sequence is ordered according to the following series:
   incisors, canines, premolars and molars,
- the given ranking order is an order from larger to smaller order, and
- the comparing means 133 calculated the four similarity measures, expressed in percentages, for a given unknown set of cranio-facial features of a human face:
   - 'choleric/strong': 25%
   - 'sanguine/dynamic': 37.5%
   - 'melancholic/delicate': 34%
   - 'phlegmatic/calm': 3.5%

In a first implementation of the example, the positioning means 135 can overlay one type of tooth with respect to one similarity measure.

In that case, the positioning means 135 may first overlay the incisors (since 'incisors' are positioned in the first position of the first predetermined sequence) with a 2D digital representation of a section through a 3D digital image of individual tooth associated with the 'sanguine/dynamic' human personality trait (since 'sanguine/dynamic' human personality trait is the largest percentage of similarity measures).

Then, in the first implementation of the example, the positioning means 135 may overlay the canines (since 'canines' are positioned in the second position of the first predetermined sequence) with a 2D digital representation of a section through a 3D digital image of individual tooth associated with the 'melancholic/delicate' human personality trait (since 'melancholic/delicate' human personality trait is the second largest percentage of similarity measures).

Further, in the first implementation of the example, the positioning means 135 may overlay the premolars (since 'premolars' are positioned in the third position of the first predetermined sequence) with a 2D digital representation of a section through a 3D digital image of individual tooth associated with the 'choleric/strong' human personality trait (since 'choleric/strong' human personality trait is the third largest percentage of similarity measures).

Finally, in the first implementation of the example, the positioning means 135 may overlay the molars (since `molars' are positioned at the last position of the first predetermined sequence) with a 2D digital representation of a section through a 3D digital image of individual tooth associated with the 'phlegmatic/peaceful' human personality trait (since `phlegmatic/peaceful' human personality trait is the latest percentage of similarity measures).

In a second implementation of the example, the positioning means 135 can overlay one or more type of tooth with respect to one similarity measure.

In particular, the number of types of tooth associated with one similarity measure can be predetermined.

In that case, the positioning means 135 may first overlay the incisors and the canines (since 'incisors' and 'canines' are positioned in the first and second positions of the first predetermined sequence) with a 2D digital representation of section through a 3D digital image of individual tooth associated with the 'sanguine/dynamic' human personality trait, respectively (since 'sanguine/dynamic' human personality traits is the largest percentage of similarity measures).

Then, in the second implementation of the example, the positioning means 135 may overlay the premolars (since 'premolars' are positioned in the third position of the first predetermined sequence) with a 2D digital representation of a section through a 3D digital image of individual tooth associated with the 'melancholic/delicate' human personality trait (since 'melancholic/delicate' human personality trait is the second largest percentage of similarity measures).

Finally, in the second implementation of the example, the positioning means 135 may overlay the molars (since 'molars' are positioned at the last position of the first predetermined sequence) with a 2D digital representation of a section through a 3D digital image of individual tooth associated with the `choleric/strong' human personality trait (since 'choleric/strong'human personality trait is the third largest percentage of similarity measures).

In that case, the positioning means 135 does not use the 'phlegmatic/calm' human personality trait for overlaying a type of tooth.

In a third implementation of the example, the positioning means 135 can overlay one type of tooth with respect to more than one similarity measure.

In that case, the positioning means 135 may first overlay the incisors (since 'incisors' are positioned in the first position of the first predetermined sequence) with a 2D digital representation of a section through a 3D digital image of individual tooth associated with the 'sanguine/dynamic' human personality trait and with the 'melancholic/delicate' human personality trait (since 'sanguine/dynamic' human personality trait and 'melancholic/delicate' human personality trait are the two largest percentages of similarity measures).

Then, in the third implementation of the example, the positioning means 135 may overlay the canines (since 'canines' are positioned in the second position of the first predetermined sequence) with a 2D digital representation of a section through a 3D digital image of individual tooth associated with the `choleric/strong' human personality trait and the 'phlegmatic/calm' human personality trait (since 'melancholic/delicate' human personality trait and the 'phlegmatic/calm'human personality trait are the smallest percentages of similarity measures).

In that case, the positioning means 135 does not use the human personality traits for overlaying the premolars and the molars. This could correspond to a situation where only the incisors and the canines need to be overlaid.

In the third implementation of the example, it was considered that, for a given type of tooth, each 3D digital image is associated with two predetermined human personality traits.

However, as explained above, for a given type of tooth, each 3D digital image may be associated with two or more predetermined human personality traits, without requiring any substantial modification of the subject application.

In a first embodiment of the subject application, the computer vision means 130 further comprise dental arch trajectory detecting means 136.

The dental arch trajectory detecting means 136 is configured for detecting a trajectory of the dental arch of the patient in the digital image of the front face of the patient.

Further, in the first embodiment of the subject application, the positioning means 135 are further configured for modifying at least one parameter (e.g., angle, width) of the dental arch trajectory of the patient, based on at least one similarity measure.

In other words, one should understand that the positioning means 135 may modify more than one parameter of the dental arch trajectory of the patient.

In a second implementation of the first embodiment of the subject application, the dental arch trajectory of the patient is modeled as a Beta function characterized by the depth and the width of the dental arch at different locations in the mouth (e.g., at the second molar region, at the canine molar region and any combination thereof).

In that case, modifying one parameter of the dental arch trajectory could correspond to modifying the depth and/or the width of the Beta function.

In a third implementation of the first embodiment of the subject application, with respect to the second implementation of the example, described above, which illustrates the functioning of the positioning means 135, the similarity measure used for modifying at least one parameter of the dental arch trajectory of the patient is one that is not used by the positioning means 135 for overlaying a type of tooth.

Further, in the first embodiment of the subject application, the positioning means 135 overlay the detected tooth with the corresponding 2D digital image or 2D digital representation of a section through the corresponding 3D digital image of individual tooth, along the modified dental arch trajectory of the patient.

In a second embodiment of the subject application, the comparing means 133 comprise a machine learning classifier.

In practice, the machine learning classifier is trained using, as training data, a plurality of set of cranio-facial features that have been extracted from a plurality of images of the front face of human faces.

Further in the second embodiment of the subject application, the machine learning classifier is configured for predicting the likelihood that the set of cranio-facial features of a human face is similar to each of the set of reference cranio-facial features of the plurality of predetermined reference cranio-facial vectors.

Yet in the second embodiment of the subject application, the machine learning classifier is further configured for outputting similarity measures representative of a matching similarity between an inputted set of cranio-facial features of a human face and each of the set of reference cranio-facial features of the plurality of predetermined reference cranio-facial vectors.

In a third embodiment of the subject application, the teeth database 111 further comprises 2D digital images of individual teeth.

In particular, each 2D digital image is associated with one type of tooth and at least one predetermined human personality trait.

In other words, one should understand that each 2D digital image may be associated with one type of tooth and more than one predetermined human personality trait.

Therefore, in the database 111, each data record corresponds to a single 2D digital image of an individual tooth which is associated with one type of tooth and at least one predetermined human personality trait.

In a first embodiment of the database 111, each data record corresponds to a single 2D digital image of an individual tooth which is associated with one type of tooth and one predetermined human personality trait.

In a second embodiment of the database 111, each data record corresponds to a single 2D digital image of an individual tooth which is associated with one type of tooth and more than one predetermined human personality trait.

This second embodiment aims at producing different tooth anatomies that extend beyond the standard tooth shapes.

In particular, in that second embodiment, each data record corresponds to a 2D digital image which is a combination of segmented portions of 2D digital images respectively associated with one predetermined human personality trait.

Indeed, each 2D digital image associated with one type of tooth and one predetermined human personality trait (such as those of the first embodiment of the database 111) may be segmented into two or more portions. Then, the segmented portions associated with one type of tooth and a plurality of predetermined human personality traits can be combined to form a combined 2D digital image. Therefore, the combined 2D digital image will be associated with the plurality of predetermined human personality traits.

Let's take an example with an incisor type of tooth.

In a first variation of the example, each 2D digital image associated with an incisor can be segmented into three portions such as a mesial portion, a distal portion and an incisal portion.

In that first variation of the example, for instance, a combined 2D digital image can correspond to a combination of a mesial segmented portion associated with a 'phlegmatic/calm' human personality trait, a distal segmented portion associated with a 'sanguine/dynamic' human personality trait and an incisal segmented portion associated with a 'melancholic/delicate' human personality trait.

However, other combinations may be contemplated, without requiring any substantial modification of the subject application.

In a second variation of the example, each 2D digital image associated with an incisor can be segmented into six portions such as a mesial cervical portion, a distal cervical portion, a mesial body portion, a distal body portion, a mesial incisal portion and a distal incisal portion.

In that second variation of the example, for instance, a combined 2D digital image can correspond to a combination of a mesial cervical segmented portion associated with a 'melancholic/delicate' human personality trait, a distal cervical segmented portion associated with a 'phlegmatic/calm' human personality trait, a mesial body segmented portion associated with a 'melancholic/delicate' human personality trait, a distal body segmented portion associated with a 'phlegmatic/calm' human personality trait, a mesial incisal segmented portion associated with a 'melancholic/delicate' human personality trait and a distal incisal segmented portion associated with a 'phlegmatic/calm' human personality trait.

However, other combinations may be contemplated, without requiring any substantial modification of the subject application.

In a variation of the first and second embodiments of the database 111, each data record may also be associated with at least one particular visual characteristic of the single 2D digital image, such as the color or the tint of the tooth.

However, other measurable characteristics of 2D digital images may be contemplated, without requiring any substantial modification of the subject application.

Further, in the third embodiment of the subject application, the memory 110 is further configured for storing a second predetermined sequence similar to the first predetermined sequence, as already described above.

In particular, the second predetermined sequence is representative of a type of tooth order, in which 2D digital images of individual teeth are to be considered for positioning on a 2D image.

Yet, in the third embodiment of the subject application, the positioning means 135 are further configured for overlaying, on the digital image of the front face of the patient, the detected tooth with 2D digital image of individual tooth, thereby generating a second personalized virtual 2D digital dentition of the patient.

In other words, one should understand that the positioning means 135 may overlay more than one 2D digital image of individual tooth.

In particular, the overlaying is based on the second predetermined sequence and the ranked similarity measures.

In an implementation of the subject application, the acquiring means 120 are further configured for acquiring a 3D intraoral scan of the dentition of the patient.

Further, in the implementation of the subject application, the teeth detecting means 131 are further configured for detecting at least one tooth in the 3D intraoral scan.

In other words, one should understand that the teeth detecting means 131 detect more than one tooth in the 3D intraoral scan.

Still further, in the implementation of the subject application, within the teeth database 111, each 3D digital image is associated with one corresponding 2D digital image.

Yet, in the implementation of the subject application, the positioning means 135 are further configured for overlaying the 3D intraoral scan with the first or second personalized virtual 2D digital dentition.

Besides, in the implementation of the subject application, the positioning means 135 are further configured for replacing the tooth detected in the 3D intraoral scan by the 3D digital image associated with the first or second personalized virtual 2D digital dentition, thereby generating a personalized virtual 3D digital dentition.

The subject application also relates to a method of training a classifier for predicting the likelihood that a set of cranio-facial features of a human face is similar to each of a set of reference cranio-facial features of a plurality of predetermined reference cranio-facial vectors.

As shown in figure 4, the method 200 is executed by a processor.

In step 210, the processor collects a plurality of set of cranio-facial training features, each being associated with at least an image of the front face of a human, as already described above.

In a first implementation of step 210, each set of cranio-facial training features comprises a combination of at least two measurable characteristic that can be derived from a substantially front face view of an individual.

In a second implementation of step 210, the collected plurality of set of cranio-facial training features comprises a plurality of positive set of cranio-facial training features and a plurality of negative set of cranio-facial training features.

In particular, in the plurality positive set of cranio-facial training features, each set of cranio-facial training features has been identified as being associated with at least one type of predetermined human personality trait.

Also, in the plurality of negative set of cranio-facial training features, each set of cranio-facial training features has been identified as not being associated with at least one type of predetermined human personality trait.

In an example of the second implementation of step 210, the number of positive set of cranio-facial training features is substantially equal to the number of negative set of cranio-facial training features.

In step 220, the processor generates a plurality of cranio-facial training vectors based on the plurality of set of cranio-facial training features, as already described above.

In step 230, the processor obtains a plurality of predetermined reference cranio-facial vectors, as already described above.

In particular, each predetermined reference cranio-facial vector is associated with at least one type of predetermined human personality trait, as already described above.

Also, each predetermined reference cranio-facial vector comprises a set of reference cranio-facial features, as already described above.

In step 240, the processor calculates a plurality of similarity measures, each being representative of a matching similarity between one set of cranio-facial training features and one set of cranio-facial training features, as already described above.

In a particular implementation of step 240, the similarity measures correspond to matching percentages.

Finally, in step 250, the processor applies the plurality of set of cranio-facial training features the plurality of similarity measures to a machine-learning algorithm to generate a trained classifier configured for outputting similarity measures representative of a matching similarity between, on the one hand, an input cranio-facial vector comprising an unknown set of cranio-facial features of a human face, and on the other hand, each of the plurality of predetermined reference cranio-facial vector.

In a first implementation of step 250, the classifier is configured for outputting the similarity measures that are greater than a predetermined similarity threshold.

In a second implementation of step 250, the machine learning algorithm comprises a machine learning algorithm selected among random forests, support vector machine and neural networks.

The subject application also relates to a computer-readable medium having stored thereon computer instructions which when executed, by a processor, perform the method 200 as described above.

The description of the subject application has been presented for purposes of illustration and description but is not intended to be exhaustive or limited to the application in the form disclosed. The embodiments were chosen and described to better explain the principles of the application and the practical application, and to enable the skilled person to understand the application for various embodiments with various modifications as are suited to the particular use contemplated.

## Claims

1. A system (100) implemented by a computer for providing personalized virtual digital dentition of a patient, the system (100) comprising:
- at least one memory (110) configured for storing
- computer program instructions,
- a plurality of predetermined reference cranio-facial vectors, each
- being associated with at least one type of predetermined human personality trait, and
- comprising a set of reference cranio-facial features,
- a teeth database (111) comprising 3D digital images of individual teeth each being associated with one type of tooth and at least one predetermined human personality trait, and
- a first predetermined sequence that is representative of a type of tooth order, in which 3D digital images of individual teeth are to be considered for positioning on a 2D image
- acquiring means (120) configured for acquiring at least one digital image of the front face of the patient smiling such that at least a portion of the dentition of the patient can be seen,
- computer vision means (130) comprising,
- teeth detecting means (131) configured for detecting at least one tooth in the digital image of the front face of the patient,
- cranio-facial detecting means (132) configured for
- detecting a set of cranio-facial features of the patient based on the digital image of the front face of the patient, and
- generating a patient cranio-facial vector based on the detected set of cranio-facial features,
- comparing means (133) configured for
- comparing the patient cranio-facial vector with the plurality of predetermined reference cranio-facial vectors, and
- calculating a plurality of similarity measures, that are greater than a predetermined similarity threshold, each being representative of a matching similarity between the patient cranio-facial vector and one predetermined reference cranio-facial vector,
- ranking means (134) configured for ranking the similarity measures according to a given ranking order, and
- positioning means (135) configured for overlaying, on the digital image of the front face of the patient, the detected tooth with a 2D digital representation of a section through the corresponding 3D digital image of individual tooth, based on the first predetermined sequence and the ranked similarity measures, thereby generating a first personalized virtual 2D digital dentition of the patient.

2. The system (100) of claim 1, wherein
- the teeth database (111) further comprises 2D digital images of individual teeth, each being associated with one type of tooth and at least one predetermined human personality trait,
- the memory (110) is further configured for storing a second predetermined sequence that is representative of a type of tooth order, in which 2D digital images of individual teeth are to be considered for positioning on a 2D image, and
- the positioning means (135) are further configured for overlaying, on the digital image of the front face of the patient, the detected tooth with the corresponding 2D digital image of individual tooth, based on the second predetermined sequence and the ranked similarity measures, thereby generating a second personalized virtual 2D digital dentition of the patient.

3. The system (100) of any one of claims 1 to 2, wherein
- the acquiring means (120) are further configured for acquiring a 3D intraoral scan of the dentition of the patient,
- the teeth detecting means (131) are further configured for detecting at least one tooth in the 3D intraoral scan,
- within the teeth database (111), each 3D digital image is associated with one corresponding 2D digital image, and
- the positioning means (135) are further configured for
- overlaying the first or second personalized virtual 2D digital dentition over the 3D intraoral scan, and
- replacing the tooth detected in the 3D intraoral scan by the 3D digital image associated with the first or second personalized virtual 2D digital dentition, thereby generating a personalized virtual 3D digital dentition.

4. The system (100) of any one of claims 1 to 3, wherein
- the computer vision means (130) further comprise dental arch trajectory detecting means (136) configured for detecting a trajectory of the dental arch of the patient in the digital image of the front face of the patient, and
- the positioning means (135) is further configured for modifying at least one parameter of the dental arch trajectory of the patient based on at least one similarity measure,
and wherein the positioning means (135) overlay the detected tooth with the corresponding 2D digital image or 2D digital representation of a section through the corresponding 3D digital image of individual tooth, along the modified dental arch trajectory of the patient.

5. The system (100) of any one of claims 1 to 4, wherein the teeth detecting means (131) are further configured for detecting a mouth opening boundary region formed between the upper lip, the lower lip, the oral commissures of the mouth of the patient,
and wherein the teeth detecting means (131) detect the tooth within the mouth opening boundary region.

6. The system (100) of claim 5, further comprising a user interface (140) configured for allowing, through user interaction, digital modification of at least one point defining the mouth opening boundary region.

7. The system (100) of any one of claims 1 to 6, wherein the comparing means (133) comprise a machine learning classifier which is trained using, as training data, a plurality of set of cranio-facial features that have been extracted from a plurality of images of the front face of human faces, the machine learning classifier being configured for
- predicting the likelihood that the set of cranio-facial features of a human face is similar to each of set of reference cranio-facial features of the plurality of predetermined reference cranio-facial vectors, and
- outputting similarity measures representative of a matching similarity between an inputted set of cranio-facial features of a human face and each of the set of reference cranio-facial features of the plurality of predetermined reference cranio-facial vectors.
